Europäisches Patentamt

European Patent Office

Office européen des brevets

Veröffentlichungsnummer: **0 292 017**
**A2**

## EUROPÄISCHE PATENTANMELDUNG

Anmeldenummer: **88108167.3**

Anmeldetag: **20.05.88**

Int. Cl.⁴: **A61K 31/725 , A61K 35/64 , A61K 35/74**

Priorität: **21.05.87 BG 79830/87**

Veröffentlichungstag der Anmeldung:
**23.11.88 Patentblatt 88/47**

Benannte Vertragsstaaten:
**BE CH DE FR GB GR IT LI NL SE**

Anmelder: **ZENTAR sa BIOGENNI STIMULATORI**
**1, Socialisticheska Pobeda**
**Sofia(BG)**

Erfinder: **Kirov, Mihail Stefanov, Dr.**
**Quartal Aprilski Block 5-A, App. 15**
**Sofia(BG)**
Erfinder: **Tzoneva, Penka Nikolova, Dr.**
**Komplex Lyulin Block 009-A**
**Sofia(BG)**

Vertreter: **von Füner, Alexander, Dr. et al**
**Patentanwälte v. Füner, Ebbinghaus, Finck**
**Mariahilfplatz 2 & 3 Postfach 95 01 60**
**D-8000 München 95(DE)**

Biostimulierendes verdauungsförderndes Mittel und Verfahren zu dessen Herstellung.

Das biostimulierende Mittel enthält lyophilisierte Zellen von Lactobacterium bulgaricum - 0,92 bis 2,68%, Pektin - 0,92 bis 2,23%, lyophilisiertes Konzentrat aus Bienen Royal Gelee - 0,37 bis 0,45% und Lösungsmittel - 94,64 bis 97,78%. Im Lösungsmittel befinden sich bis 30% Versüßungsmittel, bis 20% Antocyanine, bis 0,10% Aromastoffe und als Rest Wasser.

Im Verfahren zur Herstellung des Mittels werden native Zellen von Lactobacterium bulgaricum, Pektin und Bienen Royal Gelee homogenisiert und vorher einzeln oder gemeinsam lyophilisiert. Die Gefriertemperatur beträgt -35°C bis -45°C, wobei der Übergang zu den Plus-Temperaturen ganz langsam erfolgt-im Rahmen von 3°C bis 6°C pro Stunde- und die Trocknung des fertigen Produkts 2 bis 4 Std. dauert. Das Produkt wird unmittelbar vor dem Einsatz in dem Lösungsmittel Sirup gelöst.

Dieses biostimulierende Mittel wird bei der Behandlung und Prophylaxe des Magen-Darm-Trakts sowie in der Diätologie und Gerontologie verwendet.

# BIOSTIMULIERENDES, VERDAUUNGSFÖRDERNDES MITTEL UND VERFAHREN ZU DESSEN HERSTELLUNG

Die Erfindung betrifft ein biostimulierendes, verdauungsförderndes Mittel und ein Verfahren zu dessen Herstellung, das in der prophylaktischen und klinischen Medizin zur Prophylaxe und Behandlung des Magen-Darm-Trakts sowie in der Diätologie und Gerontologie Verwendung findet.

Es ist ein Mittel zur Behandlung von Gastritis, Kolitis und Magengeschwüren, das lyophylisierte und getrocknete Bakterienkörper von Lactobacterium bulgaricum in Nährstoff und Saccharose enthält, bekannt (Handbuch für Arzneimittel -Sofia, "Medizina i fiskultura",1982, IV Ausgabe, S. 334 (Gastropharm), S. 127 (Tagamet), S. 518 (Neutrazid).

Es ist ferner ein Mittel zur Behandlung des Magen-Darm-Trakts bekannt, das N-Cyano-N-methyl-N-2-·5-methyl-imidazol-4-yl/methyl-thioethylguanidin enthält.

Nachteil hierbei sind die unangenehmen Nebenwirkungen, wie Ekelerregung, Brechen, Juckreiz und Gynekomatie.

Es ist auch ein weiteres Mittel bekannt, das Wismuthsubnitrat, Magnesiumcarbonat, Natriumhydrogencarbonat, Cortex fragnulae und Raisoma calami enthält (Handbuch für Arzneimittel -Sofia, "Medizina i fiskultura", 1982, IV Ausgabe, S. 334 (Gastropharm), S. 127 (Tagamet), S. 518 (Neutrazid). Als Nachteil dieses Arzneimittels gilt sein streng differenziertes Verhalten zu den Erkrankungen des Magen-Darm-Trakts, namlich Neutralisierung der erhöhten Azidität der Magensäfte.

Als gemeinsamen Nachteil der bekannten Mittel gilt die Tatsache, daß keines dieser Mittel das Problem der eintretenden allgemeinen Zerrüttung und Verstimmung im Magen-Darm-Trakt auf komplexe Weise lösen kann. Ferner tragen sie nicht zur Verbesserung der Verdauuungsfunktion und zur Eliminierung der Ballaststoffe nach fortdauerndem Einnehmen von Arzneimitteln und von schwer verdaulichen Nährstoffen bei.

Der Erfindung liegt die Aufgabe zugrunde, ein biostimulierendes, verdauungsförderndes Mittel sowie auch ein Verfahren zu dessen Herstellung zu entwickeln, wobei dieses Mittel leicht verträglich sein soll, keine Nebenwirkungen hervorruft und ein komplexes Lösen des Problems bei konkreten Erkrankungen sowie hinsichtlich der eintretenden allgemeinen Verstimmung im Magen-Darm-Trakt erlaubt und weiterhin zur schnellen Beseitigung von toxischen Bestandteilen eingenommener Arzneimittel und Nährstoffe mitwirkt.

Diese Aufgabe wird durch ein Mittel gelöst, das lyophilisierte Zellen von Lactobacterium bulgaricum - 0,92% bis 2,68%. Pektin - 0,92% bis 2,23%, lyophilisiertes Konzentrat aus Bienen Royal Gelee - 0,37% bis 0,45% und Lösungs mittel - 94,64 bis 97,78% enthält.

Das eingesetzte Lösungsmittel enthält Süßstoffe bis 30%, Antocyane bis 0,20%, Aromastoffe bis 0,10% und Wasser von 70% bis 100%.

Das erfindungsgemäße Verfahren besteht darin, daß man native Zellen von Lactobacterium bulgaricum, Pektin und natives Konzentrat aus Bienen Royal Gelee homogenisiert, wobei diese Bestandteile vorher einzeln lyophilisiert oder Lactobacterium bulgaricum gemeinsam mit mindestens einem der anderen Bestandteile lyophilisiert wurden. Die Schicht des Gefrierproduktes beträgt 0,3 cm bis 0,5 cm, die Gefriertemperatur -35°C bis - 45°C und die Gefrierzeit 1,5 bis 2,5 Std. Der Übergang von der Minus- zur Plus-Temperatur erfolgt allmählich, wobei die Temperaturänderung im Intervall von 3° bis 6°C pro Stunde erfolgen soll. Die Lyophilisierung wird bei einer Temperatur von 35°C bis 40°C beendet. Das Trocknen dauert 2 bis 4 Stunden. Der Gesamtzyklus der Lyophilisierung beträgt 24 bis 30 Std.

Das so erhaltene, pulverförmige Gemisch wird unmittelbar vor Gebrauch im Lösungsmittel gelöst.

Die aktiven Bestandteile des Mittels komplettieren und stimulieren die nützliche Mikroflora. Das Präparat enthält selektive Aktivatoren der Laktanfunktion und der Enzymenprozesse und gleichzeitig normalisiert es die disbakteriosen und Magenverstimmungsabweichungen.

Das Mittel trägt zur Beseitigung der Ballaststoffe aus der normalen Verdauung sowie der Produkte, die sich beim Abbau der Arzneimittel und als Abfallprodukte bei Intoxierungsprozessen bilden, bei.

Die Verdauungswirkung des Mittels erleichtert die Beseiti gung der Abbauprodukte bei der Behandlung von Neoplasmaprozessen, bei fortdauernder Einnahme von Antibiotica und von anderen mehr oder weniger toxischen Stoffen. Es stimuliert auch die Peristaltik des Darmes.

Beispiel 1

Lactobacterium bulgaricum    - 1,89%
Pektin    - 1,51%
Bienen Royal Gelee    - 0,38%
Lösungsmittel    - 96,22%

Aufbereitung der pulverförmigen Mischung:

In eine Mischvorrichtung werden eine lyophilisierte Kultur von Lactobacterium·bulgaricum, Pektinpulver und lyophilisiertes Bienen Royal Gelee eingesetzt. Das Gemisch wird während 15 bis 20 Minuten homogenisiert und danach einer Dosiervorrichtung in einer automatischen Produktionsstraße zugegeben.

Aufbereitung des Lösungsmittels:

In einem sterilen Gefäß werden unter Umrühren 6 l einer Lösung mit einem Gehalt an 0,15% Antocyanin, 600 g Fruktose und 6 ml naturreinem Fruchtsirup gemischt. Das fertige Lösungsmittel wird einem Verteiler zum Dosieren in Fläschchen zugeleitet. Die vollen Fläschchen werden mit nach innen gewölbten Deckelchen verschlossen, in welche je 0,25 g des homogenisierten Pulvergemisches eindosiert ist. Nach Einsatz von Pfropfenbohrern werden die Fläschchen durch Metalldeckel verschlossen.

Beispiel 2

Aufbereiten des Pulvergemisches:

In ein Glasgefäß wird 1 l Kulturflüssigkeit von Lactobacterium bulgaricum mit einer Konzentration von 12,5% an Lactobacterium bulgaricum eingegossen. Unter kontinuierlichem Rühren werden dazu 0,193 l einer 13%-igen Lösung von nativem Bienen Royal Gelee und 162,5 g Pektinpulver hinzugefügt, das langsam in Portionen eingesetzt wird. Das Umrühren wird bis zur vollständigen Homogenisierung der Mischung fortgesetzt. Die Mischung wird in Pfannen aus korrosionsbeständigem Stahl in einer sehr dünnen Schicht eingegossen, nicht dicker als 0,5 cm, und dann unmittelbar bis -35°C gefroren. Das Einfrieren dauert 2 Std. Das Produkt wird bei dieser Temperatur noch für zwei Stunden stehengelassen und dann der Sublimator eingeschaltet. Man muß aufpassen, daß der Übergang von Minus- auf Plus-Temperaturen ganz langsam - im Rahmen von 3°C bis 6°C pro Stunde - erfolgt. Das Trocknen wird bei Erreichen von 35°C beendet. Diese Temperatur wird während der letzten 4 Stunden aufrechterhalten. Die ganze Lyophilisierung dauert insgesamt 24 Std. Die lyophilisierte Substanz wird durch ein Sieb mit einer Porengröße von 0,5 mm in einen hermetischen Behälter überführt in Abwesenheit von jeglicher Feuchtigkeit. Danach wird sie einer automatischen Produktionsstraße zum Dosieren in die Behälter zugeleitet.

Aufbereiten des Lösungsmittels:

Zu 10 l von Siropus Simplex werden unter Rühren 20 g Antocyanin in Pulverform und 10 ml naturreiner Fruchtsirup zugefügt. Das so erhaltene Lösungsmittel wird zum Dosieren in Fläschchen geleitet.

**Ansprüche**

1. Biostimulierendes, verdauungsförderndes Mittel, dadurch **gekennzeichnet,** daß es folgende Bestandteile in Prozent enthält:
- lyophilisierte Zellen von Lactobacterium bulgaricum - 0,92 bis 2,68%;
- Pektin - 0,92 bis 2,23%;
- lyophilisiertes Konzentrat von Bienen Royal Gelee - 0,37 bis 0,45%; und
- Lösungsmittel - Rest auf 100%, d.h. 94,64 bis 97,78%.

2. Biostimulierendes Mittel nach Anspruch 1, dadurch **gekennzeichnet,** daß das Lösungsmittel Sirup, Süßstoffe bis 30%, Antocyanine bis 0,20%, Aromastoffe bis 0,10% und Wasser von 70 bis 100% enthält.

3. Verfahren zur Herstellung eines biostimulierenden, verdauungsfördernden Mittels, dadurch **gekennzeichnet,** daß man native Zellen von Lactobacterium bulgaricum, Pektin und naturreines Konzentrat aus Bienen Royal Gelee homogenisiert, wobei die Bestandteile vorher getrennt lyophilisiert oder Lactobacterium bulgaricum und mindestens einer der anderen Bestandteile gemeinsam lyophilisiert wurden und die 0,3 bis 0,5 cm dicke Schicht des gefrorenen Produkts bei Temperaturen von -35°C bis -45°C für eine Zeitdauer von 1,5 bis 2,5 Std. gehalten wurde, wobei der Übergang von Minus- zu Plus-Temperaturen ganz langsam im Rahmen von 3°C bis 6°C pro Stunde erfolgte und die Lyophilisierung bei Temperaturen von 35°C bis 40°C endete und danach das Produkt während 2 bis 4 Std. getrocknet wurde, wo bei das so erhaltene Gemisch unmittelbar von dem Einsatz im Lösungsmittel Sirup gelöst wird.